(19) Europäisches Patentamt
European Patent Office
Office européen des brevets

(11) Veröffentlichungsnummer: **0 594 156 A1**

(12) # EUROPÄISCHE PATENTANMELDUNG

(21) Anmeldenummer: **93116991.6**

(22) Anmeldetag: **20.10.93**

(51) Int. Cl.5: **C07C 211/50**, C07C 209/68, C08G 18/10, C08G 18/32, C08K 5/18, C09D 7/12, C08G 59/50

(30) Priorität: **21.10.92 CH 3264/92**

(43) Veröffentlichungstag der Anmeldung:
**27.04.94 Patentblatt 94/17**

(84) Benannte Vertragsstaaten:
**AT BE CH DE DK ES FR GB IE IT LI NL SE**

(71) Anmelder: **LONZA AG**
**CH-3945**
**Gampel/Wallis(CH)**

(72) Erfinder: **Hardt, Peter Dr.**
**Bäretstrasse 8**
**CH-Visp (kanton Wallis)(CH)**
Erfinder: **Daum, Ulrich Dr.**
**Auf den Felsen 29**
**CH-Hofstetten (Kanton Solothurn)(CH)**
Erfinder: **Völker, Theodor**
**Rue du Nord 5**
**CH-Marly (Kanton Fribourg)(CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

(54) **Verfahren zur Herstellung von 4,4'-(Phenylendiisopropyl)-bis-(2,6-dialkylanilinen).**

(57) Es wird ein neues Verfahren zur Herstellung von 4,4'-(Phenylendiisopropyl)-bis(2,6-dialkylanilinen) der allg. Formel

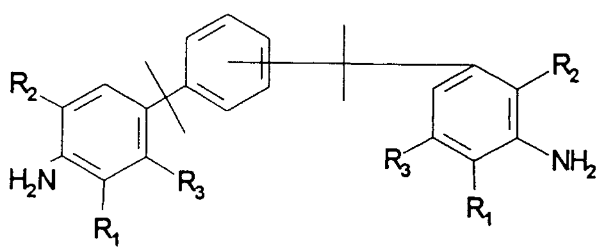

I

durch Umsetzung von einem entsprechenden Diisopropylbenzol mit einem entsprechenden Anilin in Gegenwart von katalytischen Mengen Aluminiumchlorid beschrieben.

Die 4,4'-(Phenylendiisopropyl)-bis(2,6-dialkylaniline) finden Verwendung als Vernetzungsmittel für Epoxidharze, als Kettenverlängerungsmittel für Polyurethane, zur Herstellung von Hydrolyseschutzmitteln für Polyurethane oder zur Herstellung von UV-Stabilisatoren für Lacke.

EP 0 594 156 A1

Die Erfindung beinhaltet ein neues Verfahren zur Herstellung von 4,4'-(Phenylendiisopropyl)bis(2,6-dialkylanilinen) der allg. Formel

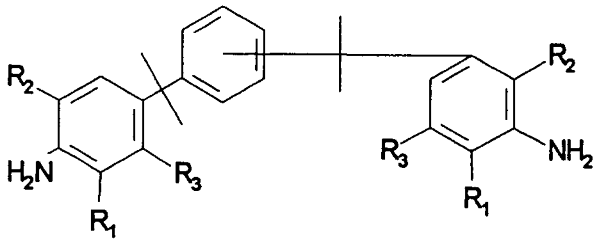

I

worin $R_1$ und $R_2$ gleich oder verschieden sein können und eine ($C_1$-$C_4$)-Alkylgruppe und $R_3$ Wasserstoff, oder ein Halogenatom bedeuten und die zentrale Phenylenfunktion in 1,3- oder 1,4-Stellung substituiert ist, ferner nicht beschriebene 4,4'-(Phenylen-1,3-diisopropyl)-bis(2,6-dialkylanilin)derivate, sowie die Verwendung der genannten Bis-aniline als Vernetzungsmittel für Epoxidharze, als Kettenverlängerungsmittel für Polyurethane, zur Herstellung vom Hydrolyseschutzmitteln für Polyurethane oder zur Herstellung von UV-Stabilisatoren für Lacke.

Eine Herstellung von 4,4'-(Phenylendiisopropyl)-bis-anilinen ist aus der holländischen Patentanmeldung 6408539 bekannt.
Beschrieben wird dort die Umsetzung von Anilin im zum Teil grossen Überschuss mit 1,4-Diisopropenylbenzol in Gegenwart von Toluol oder Benzol als Lösungsmittel und Aktivtonerden als Katalysatoren. Neben dem notwendigen grossen Anilinüberschuss erweist sich die sehr lange Umsetzungsdauer von ca. 16 Stunden für ein technisches Verfahren als grosser Nachteil. Ausserdem sind die Aktivtonerdekatalysatoren keine wohldefinierten Verbindungen, so dass die Reproduzierbarkeit der Umsetzungen problematisch ist.

Eine weitere Variante für die Herstellung von 4,4'-(Phenylendiisopropyl)-bisanilinen wird in der US Patentschrift 3200152 offenbart. Insbesondere aus Beispiel 5 geht hervor, dass die Umsetzung von Anilin mit 1,3 Diisopropylenbenzol in Gegenwart von Anilin-HCl in weniger als einer Stunde Reaktionszeit, bei einer Ausbeute von sehr guten 92%, erfolgen soll. Eigene Versuche haben jedoch gezeigt, dass diese Variante für die Umsetzung von 2,6-Dialkylanilinen unbrauchbar ist.

Im Hinblick darauf, dass die Verfahren aus dem Stand der Technik die genannten Nachteile aufweisen, bestand die Aufgabe darin, ein einfaches, wirtschaftliches und technisch gangbares Verfahren zu entwickeln.

Die Aufgabe konnte auf überraschend einfache Weise mit dem erfindungsgemässen Verfahren nach Patentanspruch 1 gelöst werden.

Die Reste $R_1$, $R_2$ und $R_3$ in den allgemeinen Formeln I, III und IV haben nachfolgende Bedeutung.

Als ($C_1$-$C_4$) Alkylgruppe für $R_1$ und $R_2$ wird zweckmässig eine Methyl-, Ethyl-, n-Propyl-, i-Propyl, n-Butyl, i-Butyl- oder t-Butylgruppe eingesetzt. In dem Fall wo $R_1$ und $R_2$ unterschiedliche Bedeutung haben, können die genannten Alkylgruppen beliebig variiert werden. Bevorzugte Kombinationen für $R_1$ ungleich $R_2$ sind Methyl und Ethyl oder Methyl und i-Propyl.
Bei gleicher Beudeutung von $R_1$ und $R_2$ Sind grundsätzlich alle der genannten Alkylgruppen einsetzbar, bevorzugt ist $R_1$ und $R_2$ Methyl, Ethyl oder i-Propyl.

$R_3$ in der Bedeutung von Halogen kann für Fluor, Chlor, Brom oder Jod stehen. Bevorzugtes Halogen ist Chlor.

Grundsätzlich kann $R_3$ in der Bedeutung von Halogen alle der genannten Kombinationen mit $R_1$ und $R_2$ eingehen. Bevorzugte Kombinationen von $R_3$ in der bevorzugten Bedeutung von Chlor mit $R_1$ und $R_2$ sind:

| | | |
|---|---|---|
| $R_1$ = Methyl | $R_2$ = Ethyl | $R_3$ = Chlor |
| $R_1$ = Ethyl | $R_2$ = Methyl | $R_3$ = Chlor |
| $R_1$ = Methyl | $R_2$ = Isopropyl | $R_3$ = Chlor |
| $R_1$ = i-Propyl | $R_2$ = Methyl | $R_3$ = Chlor |
| $R_1$ = Methyl | $R_2$ = Methyl | $R_3$ = Chlor |
| $R_1$ = Ethyl | $R_2$ = Ethyl | $R_3$ = Chlor |
| $R_1$ = Isopropyl | $R_2$ = Isopropyl | $R_3$ = Chlor |

Ausgangsprodukt ist das Diisopropenylbenzol der allgemeinen Formel

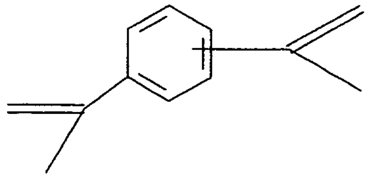

II

welches zweckmässig in einem leichten Überschuss von 10% bis 100% bezogen auf das Anilin der allgemeinen Formel

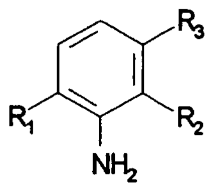

III

worin $R_1$, $R_2$ und $R_3$ die genannte Bedeutung haben, eingesetzt wird.

Aluminiumchlorid wird in katalytischen Mengen von vorzugsweise 0,02 mol bis 0,3 mol, bevorzugt 0,05 mol bis 0,20 mol bezogen auf 1 mol eingesetztes Anilin der allgemeinen Formel III eingesetzt.

Die Umsetzung erfolgt vorteilhaft unter Ausschluss von Wasser, ohne zusätzliches Lösungsmittel bei einer Reaktionstemperatur von 120 ° C bis 200 ° C vorzugsweise 140 ° C bis 160 ° C.

Gewünschtenfalls kann ein zusätzliches inertes Lösungsmittel Zugesetzt werden.

Dies bringt aber in der Regel keine Vorteile.

Die Reaktion erfolgt sehr rasch und ist in der Regel nach maximal 2 Stunden beendet. Das gewünschte 4,4'-(Phenylendiisopropyl)-bis(2,6-dialkylanilin) kann anschliessend auf fachmännische Weise aus dem Reaktionsgemisch isoliert werden.

Nach dem erfindungsgemässen Verfahren sind die bisher nicht beschriebenen 4,4'-(Phenylen-1,3-diisopropyl)-bis(2,6-dialkylaniline) der allgemeinen Formel

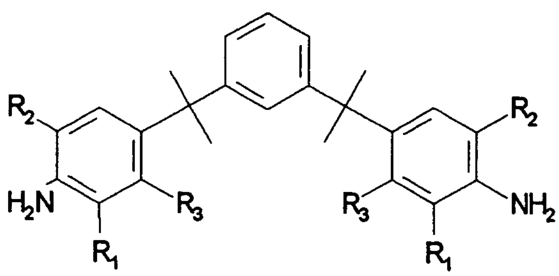

IV

worin $R_1$, $R_2$, und $R_3$ die genannte Bedeutung haben zugänglich.

Für die Bedeutung der Reste $R_1$, $R_2$ und $R_3$ wird auf die eingangs beschreibenen Definitionen verweisen.

Besonders bevorzugte Vertreter der 4,4'-(Phenylen-1,3-diisopropyl)-bis(2,6-dialkylaniline) der allgemeinen Formel IV sind:

4,4'-(Phenylen-1,3-diisopropyl)-bis(2,6-diisopropylanilin),
4,4'-(Phenylen-1,3-diisopropyl)-bis(2-isopropyl-6-methylanilin),
4,4'-(Phenylen-1,3-diisopropyl)-bis(2-ethyl-6-methylanilin),
4,4'-(Phenylen-1,3-diisopropyl)-bis(2,6-diethylanilin),
4,4'-(Phenylen-1,3-diisopropyl)-bis(3-chlor-2,6-diethylanilin)

Die einzelnen Verbindungen zeichnen sich durch ihre Vielseitigkeit in ihrer Verwendung für Spezialpolymere wie z. B. für Epoxide und Polyurethane aus.

Sowohl die Phenylen-1,3-diisopropyl- als auch die Phenylen-1,4-diisopropylderivate eignen sich gut als Vernetzungsmittel in Epoxiden, als Kettenverlängerungsmittel in Polyurethanen, nach üblicher Umsetzung

zu den entsprechenden Carbodiimiden als Hydrolyseschutzmittel für Polyurethane und nach üblicher Umsetzung zu den entsprechenden Oxalsäureamidderivaten als UV-Stabilisatoren in Lacken.

Beispiel 1

Herstellung von 4,4'-(Phenylen-1,3-diisopropyl)-bis(2,6-diisopropylanilin)
In einen 500 ml-Reaktionsgefäss mit Flügelrührer, Thermometer, Rückflusskühler und Tropfrichter wurden bei Ausschluss von Feuchtigkeit nacheinander 88,69 g 2,6-Diisopropylanilin (0,5 mol) und 6,69 g Aluminiumchlorid (0,05 mol) gegeben. Die Mischung wurde auf 1 55°C erwärmt und über den Tropftrichter während 2 Stunden mit 79, 15 g 1,3-Diisopropenylbenzol (0,5 mol) versetzt. Man liess abkühlen, versetzte mit 200 ml Hexan und 80 ml Natronlauge (20%) und liess solange rühren bis nach ca. 60 Minuten zwei klare Phasen entstanden waren. Nach Trennung der Phasen wurden die obere Hexanphase in einem 1 l-Reaktionsgefäss nach Zugabe von 300 ml Hexan mit HCl-Gas gesättigt, der ausgefallene Niederschlag auf einer Nutsche gesammelt, gut abgepresst und in 300 ml Toluol aufgeschlemmt. Man gab vorsichtig 200 ml Natronlauge (20%) hinzu und erhielt 2 klare Phasen. Die abgetrennte Toluolphase wurde durch Vakuumdestillation vom Lösungsmittel befreit. Aus dem gelbbraunen, zum Teil kristallinen Eindampfrückstand wurde durch Flashdestillation (210°C/0,2 mbar) 105,31 g zunächst flüssiges dann rasch erstarrendes 4,4'-(Phenylen-1,3-diisopropyl)-bis (2,6-diisopropylanilin) erhalten.
Ausbeute 82,1%.
Smp.: 107 - 108°C (Ethanol)

| | |
|---|---|
| $^1$HNMR: ($CDCl_3$. 300 MHz) in ppm | 1,20, d, 24H, J = 6,9 Hz; |
| | 1,61, s, 12H; |
| | 2,90, sept, 4H, J = 6,9 Hz; |
| | 3,59, s, 4H; |
| | 6,88, s. 4H; |
| | 6,99, m, 2H; |
| | 7,10, m, 1H; |
| | 7,19, m, 1H |

Beispiel 2

Herstellung von 4,4'-(Phenylen-1,3-diisopropyl)-bis(2-isopropyl-6-methylanilin)
Wie in Beispiel 1 wurden 25,50 g 2-Isopropyl-4-methylanilin (0,17 mol) und 2,09 g Aluminiumchlorid (0,016 mol) auf 155°C erwärmt und während 40 Minuten mit 15,81 g Diisopropenylbenzol versetzt. Nach Abkühlung auf 80°C, Zusatz von 250 ml Hexan und 50 ml Natronlauge (10%) wurde bei 55 - 60°C solange gerührt bis zwei klare Phasen entstanden waren. Aus der abgetrennten Hexanphase fiel beim Abkühlen und Stehenlassen bei 0°C ein weisses Kristallisat aus. Nach Abnutschen und Trocknen wurden 29,55 g weisses kristallines 4,4'-(Phenylen-1,3-diisopropyl)-bis(2-isopropyl-4-methylanilin) erhalten.
Ausbeute 76, 1%.
Smp.: 89 - 90°C

| | |
|---|---|
| $^1$HNMR: ($CDCl_3$, 300 MHz) in ppm | 1,19, d, 12H, J = 6,9 Hz; |
| | 1,60, s, 12H; |
| | 2,13, s, 6H; |
| | 2,88, sept, 2H, J = 6,9 Hz; |
| | 3,47, s, 4H; |
| | 6,77, d, 2H, J = 2,3 Hz; |
| | 6,87, d, 2H, J = 2,3 Hz; |
| | 7,00, m, 2H; |
| | 7,11, m, 1H; |
| | 7,22, m, 1H |

Beispiel 3

Herstellung von 4,4'-(Phenylen-1,3-diisopropyl)-bis(2-ethyl-4-methylanilin) 6,76 g 2-Ethyl-6-methylanilin (0,05 mol) und 0,67 g Aluminiumchlorid (0,005 mol) wurden bei 1 60°C während 30 Minuten mit 4,76 g 1,3-Diisopropenylbenzol (0,030 mol) versetzt und entsprechend Beispiel 2 aufgearbeitet. Man erhielt 7,81 g 4,4'-(Phenylen-1 ,3-diisopropyl)-bis(2-ethyl-4-methylanilin). Ausbeute 72,9%.
Smp.: 114- 115°C

1HNMR: (CDCl3, 300 MHz) in ppm 1,17, t, 6H, J = 7,5 Hz;
1,60, s, 12H;
2,12, s, 6H;
2,47, q, 4H, J = 7,5 Hz;
3,47, s, 4H;
6,77, s, 4H;
7,01, m, 2H;
7,12, m, 1H;
7,24, m, 1H

Beispiel 4

Herstellung von 4,4'-(Phenylen-1,3-diisopropyl)-bis(2,6-diethylanilin) 14,95 g 2,6-Diethylanilin (0,10 mol) wurden mit 1,40 g Aluminiumchlorid vermischt, auf 1 50 °C erwärmt und während 20 Minuten mit 9,68 g 1,3-Diisopropenylbenzol (0,061 mol) versetzt. Nach dem Abkühlen auf 60 °C wurde nach dem Versetzen mit 500 ml Hexan und 20 ml Natronlauge (20%) bei dieser Temperatur solange gerührt bis zwei klare Phasen entstanden waren. Der beim Abkühlen ausgefallene kristalline Niederschlag wurde abgetrennt und getrocknet. Man erhielt 17,66 g weisses kristallines 4,4'-(Phenylen-1,3-diisopropyl)-bis(2,6-diethylanilin)
Ausbeute 77,3%.
Smp.: 118 - 119 °C
$^1$HNMR: ($CDCl_3$, 300 MHz) in ppm 1,18, t, 12H, J = 7,5 Hz;
1,61, s, 12H
2,48, q, 8H, J = 7,5 Hz;
3,51, s, 4H;
6,79, s, 4H;
7,01, m, 2H;
7,12, m, 1H;
7,24m, 1H.

Beispiel 5 (Vergleichsbeispiel)

Gemäss US 3200152, Example 5 wurden 160,3 g 2,6-Diisopropylanilinhydrochlorid (0,75 mol), 44,3 g 2,6-Diisopropylanilin (0,25 mol) und 39,6 g 1,3-Diisopropenylbenzol (0,25 mol) während 40 Minuten auf 180 - 230 °C erwärmt. In der nach Zugabe von Natronlauge erhaltenen organischen Phase waren nur 2,6-Diisopropylanilin und 1,3-Diisopropenylbenzol, aber kein 4,4'-(Phenylen-1,3-diisopropyl)-bis(2,6-diisopropylanilin) nachweisbar.

Beispiel 6

Herstellung von 4,4'-(Phenylen-1,4-diisopropyl)-bis(2,6-diethylanilin)

15,02 g 2,6-Diethylanilin (010 mol), 1,58 g Aluminiumchlorid (0,012 mol) und 10 ml Heptan wurden auf 80 °C erwärmt und bei dieser Temperatur während 10 Minuten mit einer Lösung von 10,80 g 1,4-Diisopropenylbenzol (0,07 mol) in 40 ml Heptan versetzt. Die erhaltene klare Lösung wurde in einen Autoklaven überführt und während 1,5 Stunden auf 150 °C erwärmt. Man liess auf ca. 60 °C abkühlen, gab den Inhalt des Autoklaven zu einer Mischung aus 200 ml Heptan und 50 ml Natronlauge (30%) und liess bei leichtem Erwärmen so lange rühren bis zwei klare Phasen entstanden waren. Die noch warme obere Heptanphase wurde abgetrennt, mit Wasser nachgewaschen und bei 0 °C 1 bis 2 Tage stehengelassen. Das ausgefallene Kristallisat wurde auf einer Nutsche gesammelt, kurz mit Heptan nachgewaschen und bei 65 °C und 20 mbar getrocknet. Man erhielt 15 g kristallines nahezu weisses 4,4'-(Phenylen-1,4-diisopropyl)-bis(2,6-diethylanilin).
Ausbeute 65,7%. Durch Rückführung der Kristallisationsmutterlauge konnte die Ausbeute auf 80 bis 85% erhöht werden.
Smp.: 115,5- 116 °C
$^1$HNMR: ($CDCl_3$, 400 MHz) in ppm 1,19t,12H,J = 7,6Hz;
1 ,63 s, 12H
2,48 g, 8H, J = 7,6 Hz
3,51 s, 4H

6,81 s, 4H
7,12 s, 4H

Beispiel 7

Herstellung von 4,4'-(Phenylen-1,4-diisopropyl)-bis(2-ethyl-6-methylanilin)

Wie in Beispiel 6 wurden 13,59 g 2-Ethyl-6-methylanilin (0,10 mol) in Gegenwart von 1,53 g Aluminiumchlorid (0,012 mol) mit 10,74 g 1,4-Diisopropenylbenzol (0,07 mol) umgesetzt. Das Produkt wurde entsprechend aufgearbeitet. Man erhielt 16,32 g kristallines nahezu weisses 4,4'-(Phenylen-1,4-diisopropyl)-bis(2-ethyl-6-methylanilin).
Ausbeute 76, 1%
Smp.: 114- 116,5 °C

| | |
|---|---|
| $^1$HNMR: ($CDCl_3$, 400 MHz) in ppm | 1,18 t, 6H, J = 7,5 Hz |
| | 2,13 s, 12H |
| | 2,48 q, 4H, J = 7,5 Hz |
| | 3,47 s, 4H |
| | 6,81 m, 4H |
| | 7,12 s, 4H |

Beispiel 8

Herstellung von 4,4'-(Phenylen-1,4-diisopropyl)-bis(2-isopropyl-6-methylanilin)

23,9 g 2-Isopropyl-6-methylanilin (0,16 mol) und 2,13 g (0,016 mol) Aluminiumchlorid wurden auf 150 °C erwärmt und während 35 Minuten mit 15,8 g 1,4-Diisopropenylbenzol (0,10 mol) versetzt. Man liess auf 50 °C abkühlen, versetzte mit 250 ml Hexan und 50 ml Natronlauge (20%) und liess rühren bis zwei klare Phasen entstanden waren. Die obere Phase wurde mit Wasser nachgewaschen. Beim Stehenlassen bei -5 bis 0 °C fiel weisses Kristallisat aus. Nach dem Abtrennen und Trocknen wurden 26,64 g 4,4'-(Phenylen-1,4-diisopropyl)-bis(2-isopropyl-6-methylanilin) erhalten.
Ausbeute 72,9 %
Smp.: 112-121 °C

| | |
|---|---|
| $^1$HNMR: ($CDCl_3$, 400 MHz) in ppm | 1,20 d, 12H, J = 6,8 Hz |
| | 1,63 s, 12H |
| | 2,13 s, 6H |
| | 2,88 s, 2H, J = 6,8 Hz |
| | 3,51 s, 4H |
| | 6,78 d, 2H, J = 2,2 Hz |
| | 6,90 d, 2H, J = 2,2 Hz |
| | 7,12 s, 4H |

Beispiel 9

Herstellung von 4,4'-(Phenylen-1,4-diisopropyl)-bis(2,6-diisopropylanilin)

Wie in Beispiel 6 wurden 17,69 g 2,6-Diisopropylanilin (0,10 mol) in Gegenwart von 1,63 g Aluminiumchlorid (0,012 mol) mit 10,75 g 1,4-Diisopropenylbenzol (0,068 mol) umgesetzt. Das Produkt wurde entsprechend aufgearbeitet. Man erhielt 13,74 g nahezu weisses 4,4'-(Phenylen-1,4-diisopropyl)-bis(2,6-diisopropylanilin).
Ausbeute 53,6%. Durch Rückführung der Mutterlauge liess sich die Ausbeute auf 75 - 80% steigern.
Smp.: 123 - 129 °C

| | |
|---|---|
| $^1$HNMR: ($CDCl_3$, 400 MHz) in ppm | 1,20 d, 24H, J = 6,8 Hz |
| | 1,62 s, 12H |
| | 2,90 s, 4H, J = 6,8 Hz |
| | 3,59s, 4H |
| | 6,89s, 4H |
| | 7,11 s, 4H |

Beispiel 10

Herstellung von 4,4'-(Phenylen-1,4-diisopropyl)-bis(2,6-dimethylanilin)

Wie in Beispiel 6 wurden 12,18 g 2,6-Dimethylanilin (0,10 mol) in Gegenwart von 1,61 9 Aluminiumchlorid (0,012 mol) mit 10,72 g 1,4-Diisopropenylbenzol (0,68 mol) umgesetzt. Das Produkt wurde entsprechend aufgearbeitet. Man erhielt 12,48 g kristallines 4,4'-(Phenylen-1,4-diisopropyl)-bis(2,6-dimethylanilin). Ausbeute 62,3%. Durch Aufarbeitung der Mutterlauge wurden weitere 5,1 g Produkt gewonnen, so dass sich die Gesamtausbeute auf 88% erhöhte.

Smp.: 149,5- 150,5 ° C

$^1$HNMR: ($CDCl_3$, 400 MHz) in ppm 1,61 s, 12H
2,12 s, 12H
3,44 s, 4H
6,80 d, 4H
7,11 s, 4H

Ausprüfung der 4 4'-(Phenylendiisopropyl)-bis(2 6-dialkylanilinen)

I. Getestete Verbindungen:

1. 4,4'-(Phenylen-1,3-diisopropyl)-bis(2-ethyl-6-methylanilin) 1,3 DIPMEA
2. 4,4'-(Phenylen-1,3-diisopropyl)-bis(2,6-diethylanilin) 1,3 DIPDEA
3. 4,4'-(Phenylen-1,3-diisopropyl)-bis(2-isopropyl-6-methylanilin) 1,3 DIPMIPA
4. 4,4'-(Phenylen-1,3-diisopropyl)-bis(2,6-diisopropylanilin) 1.3 DIPDIPA
5. 4,4'-(Phenylen-1,4-diisopropyl)-bis(2,6-diethylanilin) 1,4 DIPDEA

II. Ausprüfung der in I genannten Verbindungen als Vernetzungsmittel in Epoxidharzen:

Die unter I genannten Verbindungen wurden in einer Menge von X Gewichtsteilen in 100 Gewichtsteilen Epoxybasisharz Epikote 828 (Shell) eingearbeitet. Das Harz wurde während 2 h bei 80 ° C und anschliessend während 6 h bei 130 ° C gehärtet. Tabelle 1 gibt die erhaltenen Ergebnisse wieder.

| | X | Tg( ° C) | Shore Härte D (DIN 53505) |
|---|---|---|---|
| 1,3 DIPMEA | 56.3 | 136 | 84 |
| 1,3 DIPDEA | 60.0 | 72 | 78 |
| 1,3 DIPMIPA | 60.0 | 139 | 86 |
| 1,3 DIPDIPA | 67.4 | 117 | 80 |
| 1,4 DIPDEA | 60.0 | 127 | 82 |
| Tg = Glasübergangstemperatur | | | |

III. Ausprüfung der in I genannten Verbindungen als Kettenverlängerungsmittel in Polyurethanen:

Die Vertreter der unter I genannten Verbindungen wurden in nachfolgenden TDI-Prepolymeren getestet.

Prepolymer A wurde auf fachmännisch übliche Weise durch Umsetzung von 1 mol des Polycaprolactons CAPA 205 (Interox) mit einem mittleren Molekulargewicht von 830 und von 2 mol 2,4 Toluendiisocyanat (TDI) hergestellt. Man erhielt ein Prepolymer mit einem Gehalt an freien NCO-Gruppen von 6 5%.

Prepolymer B wurde entsprechend Prepolymer A bereitet. Lediglich das Polycaprolacton wurde durch 1 mol des Polytetrahydrofurans Terathane® 1000 (Du Pont) mit einem mittleren Molekulargewicht von 1000 ersetzt. Man erhielt ein Prepolymer mit einem Gehalt an freien NCO-Gruppen von 6.3%.

Die Prüfkörper wurden durch Umsetzung von 100 Gewichtsteilen des Prepolymeren A bzw. B mit X Gewichtsteilen der entsprechenden Verbindung aus I bei ca. 60 ° C hergestellt.

Tabelle 2 gibt die erhaltenen Ergebnisse für Prepolymer A, Tabelle 3 für Prepolymer B wieder

Tabelle 2:

| | X | Shore Härte D DIN 53505 | Bruchdehnung [%] u.a. DIN 53360 | Zugfestigkeit [$N/mm^2$] DIN 53455 | Weiterreissfestigk. [N/mm] u.a. DIN 53356 |
|---|---|---|---|---|---|
| 1,3 DIPDEA | 35.5 | 50 | 304 | 33.94 | 81 |
| 1,3 DIPMIPA | 37 | 55 | 299 | 31.3 | 101 |

Tabelle 3:

| | X | Shore Härte D DIN 53505 | Bruchdehnung [%] u.a. DIN 53360 | Zugfestigkeit [$N/mm^2$] DIN 53455 | Weiterreissfestigk. [N/mm] u.a. DIN 53356 |
|---|---|---|---|---|---|
| 1,3 DIPDEA | 34.5 | 43 | 484 | 49.12 | 68 |
| 1,3 DIPDIPA | 40 | 52 | 319 | 35.22 | 80 |
| 1,3 DIPMIPA | 34 | 60 | 452 | 39.70 | 68 |

## Patentansprüche

1. Verfahren zur Herstellung von 4,4'-(Phenylendiisopropyl)-bis(2,6-dialkylanilinen) der allgemeinen Formel

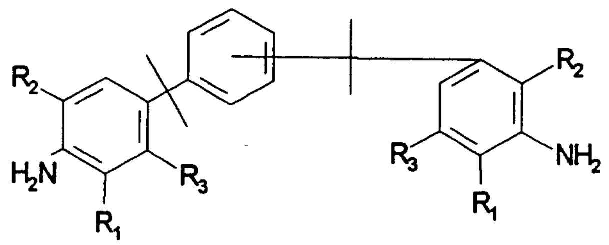

I

worin $R_1$ und $R_2$ gleich oder verschieden sein können und eine $(C_1-C_4)$-Alkylgruppe und $R_3$ Wasserstoff, oder ein Halogenatom bedeuten und die zentrale Phenylenfunktion in 1,3- oder 1,4-Stellung substituiert ist, dadurch gekennzeichnet, dass ein Diisopropenylbenzol der allgemeinen Formel

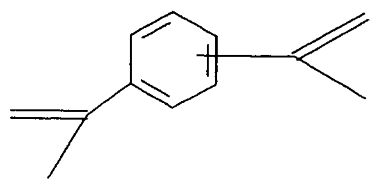

II

worin die Isopropenylgruppen am aromatischen Kern in 1,4- oder 1,3-Stellung zueinander stehen in Gegenwart von Aluminiumchlorid als Katalysator mit einem Anilin der allgemeinen Formel

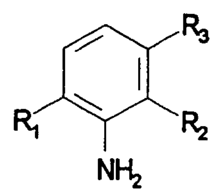

III

worin $R_1$, $R_2$ und $R_3$ die genannte Bedeutung haben, umgesetzt wird.

2. Verfahren nach Patentanspruch 1, dadurch gekennzeichnet, dass die Umsetzung unter Ausschluss von Wasser bei einer Temperatur zwischen 120 °C und 200 °C durchgeführt wird.

3. Verfahren nach einem der Patentansprüche 1 oder 2, dadurch gekennzeichnet, dass das Aluminiumchlorid in einer Menge von 0,02 mol bis 0,3 mol bezogen auf 1 mol Anilin der allgemeinen Formel III eingesetzt wird.

4. Verfahren nach einem der Patentansprüche 1 bis 3, dadurch gekennzeichnet, dass das Diisopropenylbenzol der allgemeinen Formel II bezogen auf das Anilin der allgemeinen Formel III in einem Überschuss von 10% bis 100% eingesetzt wird.

5. 4,4'-(Phenylen-1,3-diisopropyl)-bis(2,6-dialkylaniline) der allgemeinen Formel

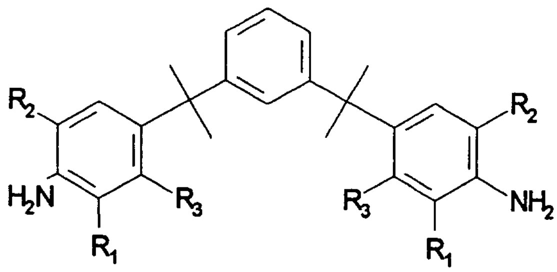

IV

worin $R_1$, $R_2$ und $R_3$ die in der allgemeinen Formel I genannte Bedeutung haben

**6.** 4,4'-(Phenylen-1,3-diisopropyl)-bis(2,6-dialkylaniline) der allgemeinen Formel IV gmäss Patentanspruch 5 worin $R_1$ und $R_2$ gleich oder verschieden sind und eine Methylgruppe, Ethylgruppe oder eine Isopropylgruppe bedeuten und $R_3$ für Wasserstoff oder ein Chloratom steht.

**7.** 4,4'-(Phenylen-1,3-diisopropyl)-bis(2,6-diisopropylanilin) als Verbindung der allgemeinen Formel IV gemäss Patentanspruch 5 mit $R_1$ und $R_2$ = Isopropyl und $R_3$ = H.

**8.** 4,4'-(Phenylen-1,3-diisopropyl)-bis(2-isopropyl-6-methylanilin) als Verbindung der allgemeinen Formel IV gemäss Patentanspruch 5 mit $R_1$ = Methyl und $R_2$ = Isopropyl und $R_3$ = H.

**9.** 4,4'-(Phenylen-1,3-diisopropyl)-bis(2-ethyl-6-methylanilin) als Verbindung der allgemeinen Formel IV gemäss Patentanspruch 5 mit $R_1$ = Methyl und $R_2$ = Ethyl und $R_3$ = H.

**10.** 4,4'-(Phenylen-1,3-diisopropyl)-bis(2,6-diethyanilin) als Verbindung der allgemeinen Formel IV gemäss Patentanspruch 5 mit $R_1$ und $R_2$ = Ethyl und $R_3$ = H.

**11.** 4,4'-(Phenylen-1,3-diisopropyl)-bis(3-chlor-2,6-diethylanilin) als Verbindung der allgemeinen Formel IV gemäss Patentanspruch 5 mit $R_1$ und $R_2$ = Ethyl und $R_3$ = Chlor.

**12.** Verwendung der 4,4'-(Phenylendiisopropyl)-bis(2,6-dialkylaniline) der allgemeinen Formel I als Vernetzungsmittel in Epoxidharzen.

**13.** Verwendung nach Patentanspruch 12 der 4,4'-(Phenylen-1,3-diisopropyl)-bis(2,6-dialkylaniline) der allgemeinen Formel IV gemäss einem der Patentansprüche 5 bis 11 als Vernetzungsmittel in Epoxidharzen.

**14.** Verwendung der 4,4'-(Phenylendiisopropyl)-bis(2,6-dialkylaniline) gemäss der allgemeinen Formel I als Kettenverlängerungsmittel in Polyurethanen.

**15.** Verwendung nach Patentanspruch 14 der 4,4'-(Phenylen-1,3-diisopropyl)-bis(2,6-dialkylaniline) der allgemeinen Formel IV gemäss einem der Patentansprüche 5 bis 11 als Kettenverlängerungsmittel in Polyurethanen.

**16.** Verwendung der 4,4'-(Phenylendiisopropyl)-bis(2,6-dialkylaniline) gemäss der allgemeinen Formel I zur Herstellung von Hydrolyseschutzmitteln für Polyurethane.

**17.** Verwendung der 4,4'-(Phenylendiisopropyl)-bis(2,6-dialkylaniline) gemäss der allgemeinen Formel I zur Herstellung von UV-Stabilisatoren für Lacke.

Europäisches Patentamt

# EUROPÄISCHER RECHERCHENBERICHT

Nummer der Anmeldung
EP 93 11 6991

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| Y | EP-A-0 492 473 (LONZA AG.)<br>* Seite 3, Zeile 13 - Zeile 44; Beispiel 12 *<br>--- | 1-4 | C07C211/50<br>C07C209/68<br>C08G18/10<br>C08G18/32<br>C08K5/18<br>C09D7/12<br>C08G59/50 |
| D,Y | US-A-3 200 152 (H. RUPPERT)<br>* Spalte 1, Zeile 13 - Spalte 2, Zeile 12 * | 1-4 | |
| X | * Spalte 2, Zeile 56 - Spalte 3, Zeile 20 * | 5-11 | |
| X | * Spalte 3, Zeile 21 - Zeile 25 *<br>--- | 12-17 | |
| D,X | US-A-3 424 795 (R.B. LUND)<br>* Spalte 1, Zeile 34 - Spalte 2, Zeile 3 *<br>* Beispiel 6 *<br>* Spalte 3, Zeile 46 - Zeile 61 *<br>--- | 5-17 | |
| A | EP-A-0 322 974 (SHELL INTERNATIONALE RESEARCH MAATSCHAPPIJ)<br>* Beispiele 2-7 *<br>--- | 12 | |
| A | US-A-4 973 754 (S.M. LI)<br>* Spalte 5, Zeile 16 - Zeile 40 *<br>----- | 1-4 | **RECHERCHIERTE SACHGEBIETE (Int.Cl.5)**<br>C07C<br>C08G<br>C08K<br>C09D |

Der vorliegende Recherchenbericht wurde für alle Patentansprüche erstellt

| Recherchenort | Abschlußdatum der Recherche | Prüfer |
|---|---|---|
| DEN HAAG | 1. Februar 1994 | Pauwels, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument